# EUROPEAN PATENT APPLICATION

(11) **EP 2 177 525 A1**
(43) Date of publication of application: **21.04.2010**
(21) Application number: 08790864.6
(22) Date of filing: 04.07.2008
(51) Int. Cl.: C07F 9/24, A61K 31/664, A61P 35/00, C07F 9/40, C07F 15/00

(54) **METAL COMPLEX COMPOUND, CANCER THERAPEUTIC COMPOSITION COMPRISING THE METAL COMPLEX COMPOUND AS ACTIVE INGREDIENT, AND INTERMEDIATE FOR PRODUCTION OF THE METAL COMPLEX COMPOUND**

(30) Priority: 06.07.2007 JP 2007179040; 15.02.2008 JP 2008034907
(71) Applicant: TMRC Co., Ltd., Tokyo 107-0052 (JP)
(72) Inventor: EKIMOTO, Hisao, Tokyo 115-0042 (JP)
(74) Representative: Jones, Helen M.M.
(86) International application number: PCT/JP2008/062133
(87) International publication number: WO 2009/008345

(57) **Abstract**

A metal complex compound which exhibits a high degree of adsorption to bone or inhibition of cell growth, and is highly effective for therapy of a cancer metastasized to bone and therapy of the primary carcinoma thereof; a therapeutic agent composition for a cancer containing as an active ingredient the metal complex compound or a physiologically acceptable salt thereof; and an intermediate for the metal complex compound are provided. More concretely, a metal complex compound represented by the following General Formula (1): 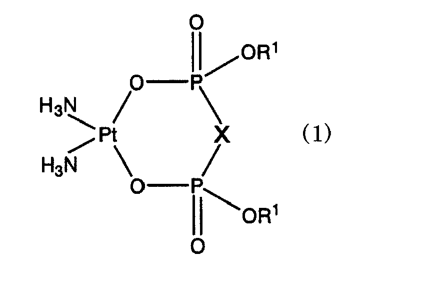 (wherein R¹ independently represents C₁-C₁₀ alkyl which may be branched or have a substituent; or a C₃-C₃₀ cyclic group which may have a substituent; and X represents CHR², an oxygen atom or NR⁵);
a therapeutic agent composition for a cancer containing it as an active ingredient; and an intermediate for the metal complex compound are provided.

## Description

### TECHNICAL FIELD

The present invention relates to a metal complex compound, a therapeutic agent composition for a cancer containing it as an active ingredient, and an intermediate for the metal complex compound. The present invention particularly relates to a novel metal complex compound active for therapy of a cancer metastasized to bone and therapy of the primary carcinoma thereof, a therapeutic agent composition for a cancer containing the metal complex compound or a physiologically acceptable salt thereof as an active ingredient, and an intermediate for the metal complex compound.

### BACKGROUND ART

Platinum complexes are known to exhibit various actions on cancer cells. For example, cisplatin is known to suppress the growth of cancer cells by binding to the double helix of DNA (Non-patent Documents 1 and 2). Further, it has been reported that therapy with such a platinum complex may cause inhibition of DNA synthesis (Non-patent Documents 3 and 4). Thus, it has been reported that a platinum complex is effective for inhibition of DNA synthesis of cancer cells growing continuously (Non-patent Documents 5 and 6).

Further, a platinum complex has an action by which the cell cycle is arrested at the G2 phase to cause apoptosis of cells, leading to their death. This apoptosis effect is observed at a lower concentration of a platinum complex than that at which the DNA synthesis-inhibition action is exerted (Non-patent Document 7), suggesting that a platinum complex acts on tumor cells by various mechanisms. Further, some types of cells exhibit a high sensitivity to a platinum complex even at the G1 phase (Non-patent Documents 8 and 9), keeping the cell cycle arrested. It is known that these actions are observed in tumors and organs in which a platinum complex is distributed at a high concentration, and that the concentration of a platinum complex distributed in bone is especially high and the duration of the actions is longer in bone.

On the other hand, bisphosphonates have been used for diseases related to bone resorption such as osteoporosis, Paget's Disease and hypercalcemia induced by a malignant tumor (Non-patent Document 10). Further, recently, they have been used for alleviation and therapy of symptoms caused by bone metastasis of breast cancer, prostate cancer, lung cancer and multiple myeloma (Non-patent Documents 11 and 12).

Further, bisphosphonates biologically act, solely or in a various state such as a conjugate, on hydroxyapatite which constitutes bone (Patent Document 1), and, for example, a synthetic compound sharing a bisphosphonate and cisplatin in its structure has been disclosed (Patent Document 2).
Patent Document 1: US Patent 6,436,386 B
Patent Document 2: WO 2005/000858 Pamphlet
Non-patent Document 1: Sherman et al., Chem. Rev., 87, 1153-81(1987)
Non-patent Document 2: Chu, J. Biol. Chem., 269, 787-90 (1994)
Non-patent Document 3: Howle et al., Biochem. Pharmacol., 19, 2757-62 (1970)
Non-patent Document 4: Salles et al., Biochem. Biophys, Res. Commun., 112, 555-63 (1983)
Non-patent Document 5: Roberts et al., Prog. Nucl. Acid Res. Mol. Biol., 22, 71-133 (1979)
Non-patent Document 6: Pinto et al., Proc. Nat. Acad. Sci.(Wash.) 82, 4616-19 (1985)
Non-patent Document 7: Soreson et al., J. Nati. Cancer Inst., 82, 749-55 (1990)
Non-patent Document 8: Krishnaswamy et al., Mutation Res., 293, 161-72 (1993)
Non-patent Document 9: Donaldson et al., Int. J. Cancer, 57, 847-55 (1994)
Non-patent Document 10: H.Fleisch., Ann. Med., 29, 55-62 (1997)
Non-patent Document 11: F.Saad et al., JNCI., 94, 1458-1468 (2002)
Non-patent Document 12: Wood. et al., J. Pharmacol. Exp. Ther., 302(3), 1055-61 (2002)

### DISCLOSURE OF THE INVENTION

### PROBLEMS TO BE SOLVED BY THE INVENTION

However, since the degree of adsorption to bone and/or the cell growth-inhibitory property of conventional metal complex compounds is/are not sufficient, a higher effect has been recently demanded in terms of therapy of a cancer metastasized to bone and therapy of the primary carcinoma thereof. Manufacture of a metal complex compound medically useful as an oral preparation has not been approved yet, and there has been no report so far on the usefulness of the compound as an oral preparation.

Thus, the present invention aims to provide a metal complex compound which exhibits a high degree of adsorption to bone or inhibition of cell growth, and is highly effective for therapy of a cancer metastasized to bone and therapy of the primary carcinoma thereof; a therapeutic agent composition for a cancer containing as an active ingredient the metal complex compound or a physiologically acceptable salt thereof; and an intermediate for the metal complex compound.

### MEANS FOR SOLVING THE PROBLEMS

The present inventor intensively studied in order to discover a compound that attains the above-described object and discovered usefulness of a certain metal complex compound, thereby completing the present invention.

That is, the metal complex compound of the present invention is represented by the following General Formula (1) : 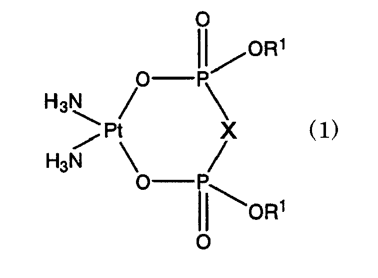 (wherein
R¹ independently represents C₁-C₁₀ alkyl which may be branched or have a substituent; or a C₃-C₃₀ cyclic group which may have a substituent; and
X represents CHR² (wherein R² represents a hydrogen atom; C₁-C₁₀ alkyl which may be branched or have a substituent; a C₃-C₃₀ cyclic group which may have a substituent; or NR³R⁴ (wherein R³ and R⁴ independently represent C₁-C₁₀ alkyl which may be branched or have a substituent; or a C₃-C₃₀ cyclic group which may have a substituent)), an oxygen atom or NR⁵ (wherein R⁵ represents C₁-C₁₀ alkyl which may be branched or have a substituent; or a C₃-C₃₀ cyclic group which may have a substituent)).

The therapeutic agent composition for a cancer of the present invention comprises as an active ingredient the metal complex compound or a physiologically acceptable salt thereof.

The intermediate for the metal complex compound of the present invention is represented by the following General Formula (2): 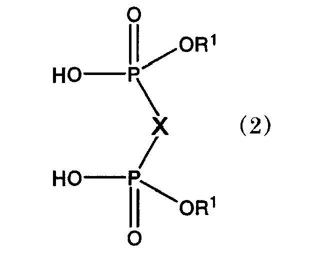 (wherein
R¹ independently represents C₁-C₁₀ alkyl which may be branched or have a substituent; or a C₃-C₃₀ cyclic group which may have a substituent; and
X represents CHR² (wherein R² represents a hydrogen atom; C₁-C₁₀ alkyl which may be branched or have a substituent; a C₃-C₃₀ cyclic group which may have a substituent; or NR³R⁴ (wherein R³ and R⁴ independently represent C₁-C₁₀ alkyl which may be branched or have a substituent; or a C₃-C₃₀ cyclic group which may have a substituent)), an oxygen atom or NR⁵ (wherein R⁵ represents C₁-C₁₀ alkyl which may be branched or have a substituent; or a C₃-C₃₀ cyclic group which may have a substituent)).

### EFFECT OF THE INVENTION

According to the present invention, a metal complex compound which exhibits a high degree of adsorption to bone or inhibition of cell growth, and is highly effective for therapy of a cancer metastasized to bone and therapy of the primary carcinoma thereof; a therapeutic agent composition for a cancer containing as an active ingredient the metal complex compound or a physiologically acceptable salt thereof; and an intermediate for the metal complex compound can be provided.

### BEST MODE FOR CARRYING OUT THE INVENTION

The present invention will be described in more detail below.

The metal complex compound of the present invention is represented by the following General Formula (1): 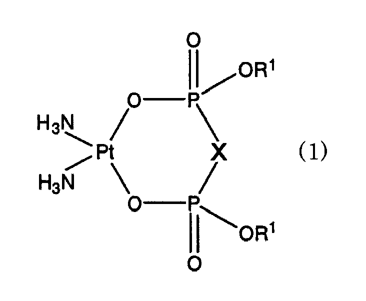 (wherein
R¹ independently represents C₁-C₁₀ alkyl which may be branched or have a substituent; or a C₃-C₃₀ cyclic group which may have a substituent; and
X represents CHR² (wherein R² represents a hydrogen atom; C₁-C₁₀ alkyl which may be branched or have a substituent; a C₃-C₃₀ cyclic group which may have a substituent; or NR³R⁴ (wherein R³ and R⁴ independently represent C₁-C₁₀ alkyl which may be branched or have a substituent; or a C₃-C₃₀ cyclic group which may have a substituent)), an oxygen atom or NR⁵ (wherein R⁵ represents C₁-C₁₀ alkyl which may be branched or have a substituent; or a C₃-C₃₀ cyclic group which may have a substituent)).

The intermediate for the metal complex compound of the present invention is represented by the following General Formula (2): 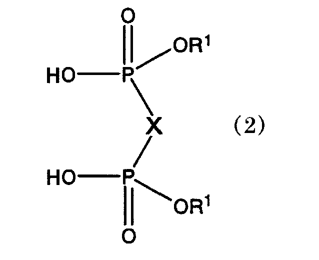 (wherein
R¹ independently represents C₁-C₁₀ alkyl which may be branched or have a substituent; or a C₃-C₃₀ cyclic group which may have a substituent; and
X represents CHR² (wherein R² represents a hydrogen atom; C₁-C₁₀ alkyl which may be branched or have a substituent; a C₃-C₃₀ cyclic group which may have a substituent; or NR³R⁴ (wherein R³ and R⁴ independently represent C₁-C₁₀ alkyl which may be branched or have a substituent; or a C₃-C₃₀ cyclic group which may have a substituent)), an oxygen atom or NR⁵ (wherein R⁵ represents C₁-C₁₀ alkyl which may be branched or have a substituent; or a C₃-C₃₀ cyclic group which may have a substituent)).

Examples of the C₁-C₁₀ alkyl groups represented by R¹, R², R³, R⁴ and R⁵ which may be branched or have a substituent in the above-described General Formulae (1) and (2) include methyl, ethyl, propyl, isopropyl, butyl, isobutyl, secondary butyl, tertiary butyl, amyl, isoamyl, tertiary amyl, hexyl, heptyl, octyl, isooctyl, 2-ethylhexyl, tertiary octyl, nonyl, decyl and the like, wherein an arbitrary -CH₂- in such alkyl groups may be substituted with -O-, -CO-, -COO- or -SiH₂-, and each of a part or all of the hydrogen atoms may be substituted by a halogen atom such as fluorine, chlorine, bromine or iodine; cyano; or -SO₂.

The C₃-C₃₀ cyclic groups represented by R¹, R², R³, R⁴ and R⁵ which may have a substituent in the above-described General Formulae (1) and (2) may be monocyclic or polycyclic, or may form a fused ring or assembled ring; and may be used irrespective of whether it is an aromatic cyclic group or a saturated aliphatic cyclic group; and each carbon atom in the ring may be substituted with an oxygen atom, nitrogen atom, sulfur atom or the like; and each of a part or all of the hydrogen atoms in the ring may be substituted with C₁-C₂₁ alkyl, C₁-C₂₁ alkoxy, amino, a halogen atom, -SO₂ or the like.

Examples of such a cyclic group include pyrrole, furan, thiophene, imidazole, pyrazole, oxazole, isoxazole, thiazole, isothiazole, pyridine, pyridazine, pyrimidine, pyrazine, piperidine, piperazine, morpholine, phenyl, naphthyl, anthracene, biphenyl, triphenyl, 2-methylphenyl(o-tolyl, cresyl), 3-methylphenyl(m-tolyl), 4-methylphenyl(p-tolyl), 4-chlorophenyl, 4-hydroxyphenyl, 3-isopropylphenyl, 4-isopropylphenyl, 4-butylphenyl, 4-isobutylphenyl, 4-tert-butylphenyl, 4-hexylphenyl, 4-cyclohexylphenyl, 4-octylphenyl, 4-(2-ethylhexyl)phenyl, 4-stearylphenyl, 2,3-dimethylphenyl(xylyl), 2,4-dimethylphenyl, 2,5-dimethylphenyl, 2,6-dimethylphenyl, 3,4-dimethylphenyl, 3,5-dimethylphenyl, 2,4-di-tert-butylphenyl, 2,5-di-tert-butylphenyl, 2,6-di-tert-butylphenyl, 2,4-di-tert-pentylphenyl, 2,5-di-tert-amylphenyl, 2,5-di-tert-octylphenyl, 2,4-dicumylphenyl, cyclohexylphenyl, biphenyl, 2,4,5-trimethylphenyl (mesityl), 4-aminophenyl, 5-dimethylaminonaphthyl, 6-ethoxy-benzothiazolyl, 2,6-dimethoxy-4-pyrimidyl, 5-methyl-1,3,4-thiadiazole-2-yl and 5-methyl-3-isoxazolyl.
In the present invention, each R¹ in the above General Formulae (1) and (2) is preferably methyl, ethyl, propyl, butyl, octyl, cyclohexyl, phenyl or benzyl.

The metal complex compound of the present invention is preferably represented by the following General Formula (3): 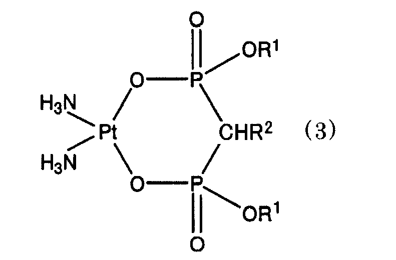 wherein R¹ and R² have the same meanings as described above. In the formula (3), R² is preferably a hydrogen atom, methyl, ethyl, propyl, butyl, octyl, benzyl or NR³R⁴ (wherein R³ and R⁴ independently represent a hydrogen atom, methyl, ethyl, propyl, butyl, octyl or benzyl). Examples of such a metal complex compound include the aminomethylenebisphosphonate platinum complex. Further, the metal complex compound of the present invention is preferably represented by the following General Formula (4): 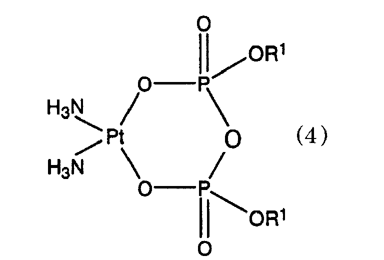 wherein R¹ has the same meaning as described above. Further, the metal complex compound of the present invention is preferably represented by the following General Formula (5): 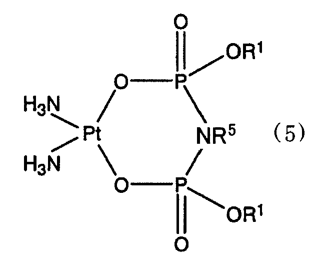 wherein R¹ and R⁵ have the same meanings as described above. In the formula (5), R⁵ is preferably a hydrogen atom, methyl, ethyl, propyl, butyl or octyl. Examples of such a metal complex compound include the imidobisphosphonate platinum complex.

The metal complex compound of the present invention represented by the above-described General Formula (1) includes, for example, metal salts of P,P'-dialkylmethylene bisphosphonic acid, metal salts of P,P'-dialkyl pyrophosphoric acid and metal salts of P,P'-dialkylimino diphosphate, and is different from the metal complex compound described in Patent Document 2 which includes metal salts of methylene bisphosphonic acid, in various aspects such as the structure, synthesis method, physical property and biological activity.

The production method of the metal complex compound of the present invention is not particularly limited, and the compound may be produced using the intermediate of the metal complex compound of the present invention represented by the above-described General Formula (2). The compound can be preferably obtained, for example, by dissolving cisplatin in dimethylformamide (DMF) and adding a P,P'-dialkylmethylene bisphosphonic acid compound and silver(I) oxide to the resulting solution, followed by stirring of the resulting mixture, filtration of the white precipitates through Celite, concentration of the filtrate and recrystallization of the residue with absolute ethanol.

The production method of the intermediate of the metal complex compound of the present invention is not particularly limited, and the intermediate can be preferably obtained, for example, by dissolving tetraethyl methylenebisphosphonate in morpholine and heating the resulting solution to reflux to produce a morpholinium salt, which morpholinium salt is then dissolved in 25 mL of methanol, followed by addition of Amberlite IR-120(H⁺) resin thereto, stirring of the resulting mixture, filtering of this solution and evaporation of the solvent under reduced pressure.

The therapeutic agent composition for a cancer of the present invention contains as an active ingredient the above-mentioned metal complex compound of the present invention or a physiologically acceptable salt thereof. This therapeutic agent composition for a cancer preferably cures a primary carcinoma, and preferably cures a cancer metastasized to bone selected from primary carcinomas. Examples of the primary carcinomas include lung cancer, gastric cancer, colon cancer, ovarian cancer, brain tumor, breast cancer, renal cancer, prostate cancer and melanoma, and, especially, breast cancer, prostate cancer, lung cancer and colon cancer are preferred since these are cancers prone to metastasize to bone and difficult to cure.

The therapeutic agent composition for a cancer of the present invention may be in various dosage forms depending on the prophylactic or therapeutic purpose, and examples of the dosage form include oral preparations, injection solutions, suppositories, ointments and patches, and oral preparations are preferred, and solid formulations for oral administration are more preferred. For example, when a solid formulation for oral administration is prepared, a vehicle and, as required, a binder, disintegrator, lubricant, coloring agent, corrective and/or the like may be added followed by production of a tablet, coated tablet, granule, powder, capsule or the like according to a conventional method. Each of these dosage forms may be produced by a formulation method known to and commonly used by those skilled in the art.

The additives may be those generally used in the art, and examples of the vehicle include lactose, saccharose, sodium chloride, glucose, starches, calcium carbonate, kaolin, microcrystalline cellulose and silicic acid; examples of the binder include water, ethanol, propanol, simple syrups, glucose solutions, starch solutions, gelatin solutions, carboxymethylcellulose, hydroxypropyl cellulose, hydroxypropyl starch, methyl cellulose, ethyl cellulose, shellac, calcium phosphate and polyvinylpyrrolidone; examples of the disintegrator include dry starch, sodium alginate, powdered agar, sodium hydrogen carbonate, calcium carbonate, sodium lauryl sulfate, stearic acid monoglyceride and lactose; examples of the lubricant include purified talc, stearate, borax and polyethylene glycols; examples of the coloring agent include titanium oxide and ferric oxide; and examples of the corrective include saccharose, orange peel, citric acid and tartaric acid.

The effective amount of the metal complex compound which is the active ingredient of the therapeutic agent composition for a cancer of the present invention is generally not limited, and required to be determined as appropriate depending on the suitable dosage form. That is, although the dose is set differently depending on the required therapeutic effect, administration route and duration of the therapeutic effect, 0.001 to 100 mg/kg/day, preferably 0.1 to 30.0 mg/kg/day with respect to the body weight is appropriate in mammals including human and others in the case of oral administration. This dose is used for single administration or multiple administrations.

### EXAMPLES

The present invention will be described more concretely by way of Examples below. The compound of the present invention is not limited to the Examples below.

### (Example 1)

### (Compound 1)

Tetraethyl methylenebisphosphonate (3.0 g, 10.4 mmol) was dissolved in morpholine (15.6 mL, 179 mmol), and the resulting solution was heated to reflux for 6 hours. After cooling the solution to room temperature, unreacted morpholine was evaporated under reduced pressure. The residues were washed with anhydrous ether (20 mL), and the obtained solids were dried under reduced pressure to obtain a morpholinium salt (Compound 1) (3.84 g, 91%) having the physical properties below.

### Morpholinium Salt (Compound 1)

Pale yellow solid; ¹H-NMR(CDCl₃) δ3.87(m, 12H), 3.14(t, J=4.9Hz, 8H), 2.08(t, J_{P}, _{H}=19.7Hz,2H), 1.26(t, J=7.0 Hz, 6H) (Compound 2)

Amberlite IR-120(H⁺) resin (9.00 g) was added to the morpholinium salt (Compound 1) (3.84 g, 9.45 mmol) dissolved in 25 mL of methanol, and the resulting mixture was stirred at room temperature for 4 hours. This solution was filtered and the solvent was evaporated under reduced pressure to obtain bisphosphonic acid (Compound 2) (2.19 g, quant.) having the physical properties below (see Gantla Vidyasagar Reddy, Albert W. Herlinger, Synthetic Communications, 2004, 34(5), 331-344).

### Bisphosphonic Acid (Compound 2)

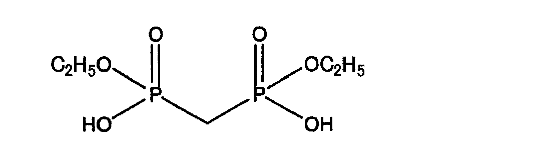

White solid; ¹H-NMR (CDCl₃) δ8.47(br, 2H), 4.13(dq, J_{P}, _{H}=8.1 Hz, J=7.1 Hz, 4H), 2. 43 (t, J_{P}, _{H}=21.5 Hz, 2H), 1.33(t, J=7.11 Hz, 6H); ¹H-NMR(CD₃OD) δ4.11(dq, J_{P}, _{H}=8.0 Hz, J=7.0 Hz, 4H), 2.46(t, J_{P}, _{H}=21.1 Hz, 2H), 1.32(t, J=7.0 Hz, 6H); ³¹P-NMR(CD₃OD) δ18.20(s); IR(KBr)ν (cm⁻¹) 979, 2902, 2350, 1647, 1206, 1043, 998, 951, 823, 506, 456; FAB-MS (m/z): (M⁺+H) 233

### (Compound 3)

Cisplatin (990 mg, 3.3 mmol) was dissolved in DMF (25 mL), and the Compound 2 (766 mg, 3.3 mmol) and silver(I) oxide (928 mg, 4.0 mmol) were added to the resulting solution, followed by stirring thereof at room temperature. The white precipitates were filtered through Celite, and the filtrate was concentrated. Residues were recrystallized with absolute ethanol to obtain the Compound 3 (242 mg, 16%) having the physical properties below.

### Compound 3

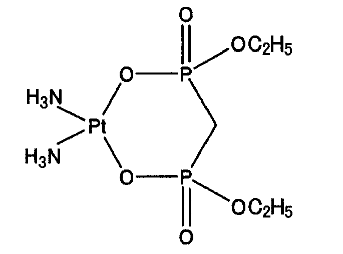

White powder; Yield: 16%; ¹H-NMR(CD₃OD) δ4.04(m, 4H), 2.28 (t, J_{P}, _{H}=19.3 Hz, 1H), 2.26 (t, J_{P}, _{H}=19.6 Hz, 1H), 1.30(t, J=7.1 Hz, 6H); ³¹P-NMR(CD₃OD) δ27.89(s); IR(KBr)ν (cm⁻¹) 3234, 3069, 1180, 1034, 944, 803, 568; HRMS-FAB (m/z): Calcd.: C₅H₁₉O₆N₂P₂Pt (M⁺+H) 460.0366, Found: 460.0354; Analysis: Calcd.: C₅H₁₈N₂O₆P₂Pt C:13.08, H:3.95, N:6.10; Found: C:12.98, H:3.99, N5.82

### (Example 2)

### (Compound 4)

Under argon stream, methylenebis(phosphonic dichloride) (2.0 g, 8.00 mmol) was dissolved in 10 mL of toluene, and the resulting solution was stirred vigorously. After dissolution of all the substances, a solution of benzylalcohol (3.31 mL, 32.0 mmol) and pyridine (2.46 mL, 30.4 mmol) dissolved in 20 mL of toluene was added dropwise to the above solution through a dropping funnel at 0°C for not less than 2 hours. After allowing the resulting mixture to warm to room temperature, it was stirred overnight, and pyrinidium salt was removed by filtration, followed by washing of the filtrate with 2 M aqueous sodium hydroxide solution (2×6 mL) and water (2×6 mL). The organic phase was dried and the solvent was then evaporated under reduced pressure. The remaining benzyl alcohol was evaporated by distillation under reduced pressure to obtain the Compound 4 (1.74 g, 41%) below (see Phillip C. B. Page, Michael J. McKenzie, and James A. Gallangher, J.Org. Chem, 2001, 66, 3704-3708).

### Compound 4

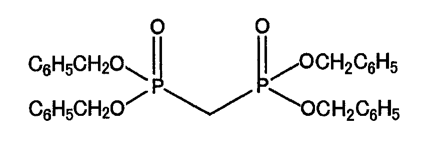

Colorless oil; ¹H-NMR(CDCl₃) δ7.30(s, 20H), 5.02(m, 4H), 2.51(t, J_{P}, _{H}=21.2 Hz, 2H)

### (Compound 5)

The Compound 5 (49%, 2 or more stages) having the physical properties below was obtained from the Compound 4 by the same operations as in the preparation of the Compound 2 (see Gantla Vidyasagar Reddy, Albert W. Herlinger, Synthetic Communications, 2004, 34(5), 331-344).

### Compound 5

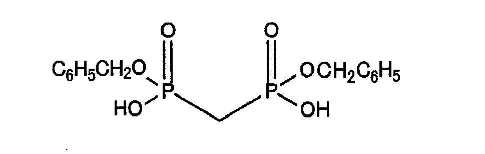

Colorless oil; ¹H-NMR(CDCl₃) δ7.36-7.320(m, 10H), 6.82(br, 2H), 5.10(d, J_{P}, _{H}=7.9 Hz, 4H) 2.45(t, J_{P}, _{H}=21.6 Hz, 2H); ³¹P-NMR(CDCl₃) δ20.06(s); 1H-NMR(CD₃OD) δ7.31(m, 10H), 5.07(d, J_{P}, _{H}=6.9 Hz, 4H), 2.54(t, J_{P}, _{H}=21.1 Hz, 2H); ³¹P-NMR(CD₃OD) δ20.08(s)

### (Compound 6)

The Compound 6 having the physical properties below was obtained from the Compound 5 by the same operations as in the preparation of the Compound 3.

### Compound 6

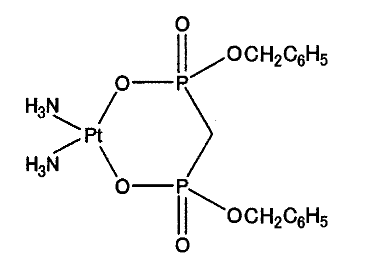

¹H-NMR(CD₃OD) δ7.48-7.20(m, 10H), 4.98(m, 4H), 2.38(t, J_{P}, _{H}=19.0 Hz, 1H), 2.34(t, J_{P}, _{H}=19.7 Hz, 1H)

### (Example 3)

### (Compound 7)

Under argon stream, methylenebis(phosphonic dichloride) (2.0 g, 8.00 mmol) and 1H-tetrazole (84.0mg, 1.20mmol) were dissolved in 80 mL of toluene, and the resulting mixture was stirred vigorously. After dissolution of all the substances, a solution of 1-octanol (2.49 mL, 15.8 mmol) and diisopropylethylamine (3.07 mL, 17.6 mmol) dissolved in 60 mL of toluene was added dropwise to the above resulting solution through a dropping funnel for not less than 2 hours. After stirring the resulting mixture at room temperature overnight, the reaction was quenched with 50 mL of water, and the mixture was stirred for 15 minutes. The reaction mixture was transferred to a separatory funnel to separate the organic phase, which was then washed twice with 0.1M HCl (60 mL). The organic phase was dried over anhydrous sodium sulfate and the solvent was then evaporated under reduced pressure to obtain the Compound 7 (1.72 g, 54%) having the physical properties below (see Dominique C. Stepinski, Derek W. Nelson, Peter R. Zalupski, AlbertW. Herlinger, Tetrahedron, 2001, 57, 8637-8645).

### Compound 7

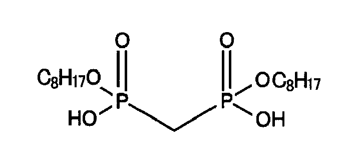

White solid; ¹H-NMR(CDCl₃) δ6.72(br, 2H), 4.04(dt, J_{P}, _{H}=J=6.7 Hz, 4H), 2. 42 (t, J_{P}, _{H}=21.8 Hz, 2H), 1. 66 (tt, J=6.8 Hz, 4H), 1.32-1.27(m, 20H), 0.88(t, J=6.8 Hz, 6H);¹H-NMR(CD₃OD) δ4.04(dt, J_{P}, _{H}=J=6.7 Hz, 4H), 2. 45 (t, J_{P}, _{H}=20.8 Hz, 2H), 1.68(tt, J=6.9 Hz, 4H), 1.40-1.31(m, 20H), 0.90(t, J=6.8 Hz, 6H) ; ³¹P-NMR (CD₃OD) δ19.83(s); FAB-MS (m/z) : (M⁺+H) 401

### (Compound 8)

The Compound 8 having the physical properties below was obtained from the Compound 7 by the same operations as in the preparation of the Compound 3.

### Compound 8

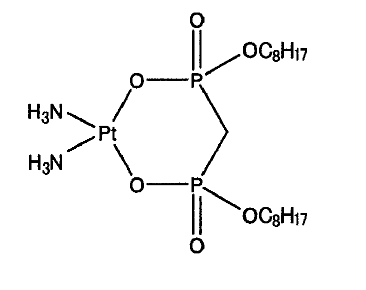

¹H-NMR(CD₃OD) δ3.97(m, 4H), 2.27(t, J_{P}, _{H}=20.4 Hz, 2H), 1.69-1.61(m, 4H), 1.41-1.33(m, 20H), 0.89(br, 6H);³¹P-NMR(CD₃OD) δ28.69 (s) ; FAB-MS (m/z) : (M⁺+H) 628

### (Example 4)

### (Compound 9)

Under argon stream, methylenebis(phosphonic dichloride) (2.0 g, 8.00 mmol) and 1H-tetrazole (84.0mg, 1.20mmol) were dissolved in 80 mL of toluene, and the resulting mixture was stirred vigorously. After dissolution of all the substances, a solution of 1-hexanol (1.98 mL, 15.8 mmol) and diisopropylethylamine (3.07 mL, 17.6 mmol) dissolved in 30 mL of toluene was added dropwise to the above resulting solution through a dropping funnel for not less than 2 hours. After stirring the resulting mixture at room temperature overnight, the reaction was quenched with 50 mL of water, and the mixture was stirred for 15 minutes. The reaction mixture was transferred to a separatory funnel to separate the organic phase, which was then washed twice with 0.1M HCl (60 mL). The organic layer was dried over anhydrous sodium sulfate and the solvent was then evaporated under reduced pressure to obtain the Compound 9 (2.32 g, 84%) having the physical properties below (see Dominique C. Stepinski, Derek W. Nelson, Peter R. Zalupski, AlbertW. Herlinger, Tetrahedron, 2001, 57, 8637-8645).

### Compound 9

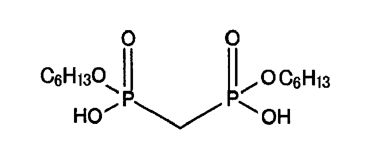

White oily solid; ¹H-NMR(CDCl₃) δ6.61(br, 2H), 4.05(dt, J_{P}, _{H}=J=6.7 Hz, 4H), 2.41(t, J_{P}, _{H}=21.5 Hz, 2H), 1.65(tt, J=6.7 Hz, 4H), 1.39-1.28(m, 12H), 0.88(t, J=6.7 Hz, 6H); ¹H-NMR(CD₃OD) δ4.04(dt, J_{P}, _{H}=J=6.6 Hz, 4H), 2.45(t, J_{P}, _{H}=21.1 Hz, 2H), 1.68(tt, J=6.7 Hz, 4H), 1.40-1.30(m, 12H), 0.91(t, J=6.9 Hz, 6H) ; ³¹P-NMR(CD₃OD) δ19.83(s); FAB-MS (m/z): (M⁺+H) 345

### (Compound 10)

The Compound 10 having the physical properties below was obtained from the Compound 9 by the same operations as in the preparation of the Compound 3.

### Compound 10

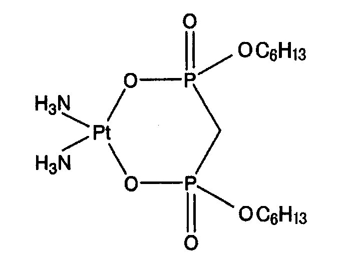

White powder; Yield: 8.7%; ¹H-NMR(CD₃OD) δ3.96(m, 4H), 2.27(t, J_{P}C _{H}=19.6 Hz, 2H), 1.65(tt, J=6.4 Hz, 4H), 1.41-1.33(m, 12H), 0.91(t, J=6.7 Hz, 6H) ;³¹P-NMR(CD₃OD) δ28.46(s); FAB-MS(m/z):(M⁺+H)572; Analysis: Calcd.: C₁₃H₃₄N₂O₆P₂Pt C:27.32, H:6.00, N:4.90; Found: C:26.17, H:5.76, N:5.00

### (Example 5)

### (Compound 11)

Under argon stream, to a solution of sodium hydride (ca. 60%) (457 mg, 11.4 mmol) suspended in 10 mL of THF (tetrahydrofuran), tetraethyl methylenebisphosphonate (3.00 g, 10.4 mmol) was added dropwise at 0°C. After stirring the resulting mixture at 0°C for 1.5 hours, 1-bromopropane (1.40 g, 11.4 mmol) dissolved in 3 mL of THF was added dropwise thereto. After allowing the resulting mixture to warm to room temperature, it was stirred overnight. After confirming by silica gel TLC that the raw material was remaining, an equal amount of 1-bromopropane was added dropwise to the reaction solution, and the resulting mixture was stirred at room temperature for 6 days. Saturated aqueous ammonium chloride solution (20 mL) was added to the reaction solution to quench the reaction. This solution was extracted with dichloromethane (3x30 mL) and the organic phase was washed with saturated brine (2×30 mL). The organic phase was dried, and the solvent was evaporated under reduced pressure. The residues were purified by silica gel chromatography (CH₂Cl₂:MeOH=98:2→9:1) to obtain the Compound 11 (1.99 g, 58%) having the physical properties below.

### Compound 11

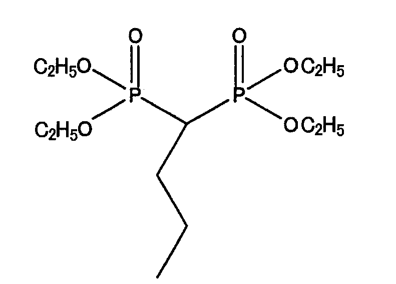

Colorless oil; ¹H-NMR(CDCl₃) δ4.24-4.11(m, 8H), 2.28(tt, J_{P}, _{H}=24.2 Hz, J=6.0 Hz, 7.3 Hz, 3H)

### (Compound 12)

The Compound 12 (99% or more, 2 or more stages) having the physical properties below was obtained from the Compound 11 by the same operations as in the preparation of the Compound 2.

### Compound 12

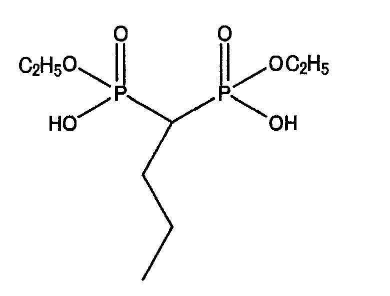

Colorless oil; ¹H-NMR(CDCl₃) δ7.96(br, 2H), 4.21-4.13(m, 4H), 2.28(tt, J_{P}, _{H}=24.5 Hz, J=5.4 Hz, 1H), 1.98-1.84(m, 2H), 1.54(sept, J=7.0 Hz, 6H), 0.91(t, J=7.3 Hz, 3H); ¹H-NMR (CD₃OD) δ4.12(dq, J_{P}, _{H}=8.9 Hz, J=7.1 Hz, 4H), 2.27(tt, J_{P}, _{H}=24.1 Hz, J=6.0 Hz, 1H), 1.95-1.81(m, 2H), 1.61(sept, J=7.5 Hz, 2H), 1.32(t, J=7.1 Hz, 6H), 0. 93 (t, J=7.3 Hz, 3H); ³¹P-NMR (CD₃OD) δ23.86(s); FAB-MS (m/z): (M⁺+H) 275

### (Compound 13)

The Compound 13 having the physical properties below was obtained from the Compound 12 by the same operations as in the preparation of the Compound 3.

### Compound 13

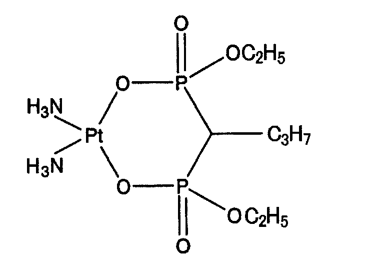

¹H-NMR(CD₃OD) δ4.17-3.96(m, 4H), 2.17-2.05(m, 1H), 1.93-1.83(m, 2H), 1.59(brs, 2H), 1.33-1.23(m, 6H), 0.92(t, J=6.8 Hz, 3H); ³¹P-NMR(CD₃OD) δ32.61(s), 30. 91 (s) ; FAB-MS(m/z): (M⁺+H) 502

### (Example 6)

### (Compound 14)

Under argon stream, to a solution of sodium hydride (ca. 60%) (305 mg, 7.63 mmol) suspended in 6.3 mL of THF, tetraethyl methylenebisphosphonate (2.00 g, 6.94 mmol) was added dropwise at 0°C. After stirring the resulting mixture at 0°C for 1.5 hours, benzyl bromide (1.31 g, 7.64 mmol) dissolved in 2 mL of THF was added dropwise thereto. After allowing the resulting mixture to warm to room temperature, it was stirred overnight. After confirming by silica gel TLC that the raw material was remaining, benzyl bromide (0.59 g, 3.47 mL) was added dropwise to the reaction solution, and the resulting mixture was stirred at room temperature for 3 days. Saturated aqueous ammonium chloride solution (15 mL) was added to the reaction solution to quench the reaction. This solution was extracted with dichloromethane (3×20 mL) and the organic phase was washed with saturated brine (2×20 mL). The organic phase was dried, and the solvent was evaporated under reduced pressure. The residues were purified by silica gel chromatography (CH₂Cl₂:MeOH=98:2) to obtain the Compound 14 (1.87 g, 71%) having the physical properties below.

### Compound 14

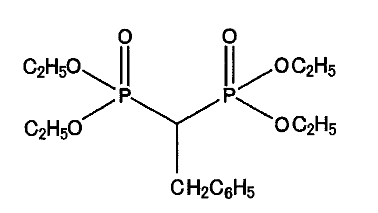

Colorless oil; ¹H-NMR(CDCl₃) δ7.28-7.18(m, 5H), 4.17-4.01(m, 8H), 3.25(dt, J=6. 3 Hz, J_{P}, _{H}=16.6 Hz, 2H), 2.65(tt, J_{P}, _{H}=23.9 Hz, J=6.3 Hz, 1H), 1.34(q, J=7.1 Hz, 12H)

### (Compound 15)

The Compound 15 (84%, 2 or more stages) having the physical properties below was obtained from the Compound 14 by the same operations as in the preparation of the Compound 2.

### Compound 15

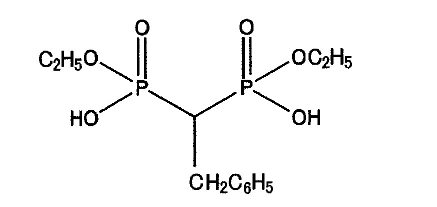

Colorless oil; ¹H-NMR(CDCl₃) δ7.29-7.18(m, 5H), 6.83(br, 2H), 4.07-4.01(m, 2H), 3.99-3.90(m, 2H), 3.26(dt, J=5.8 Hz, J_{P}, _{H}=16.7 Hz, 2H), 2.65(tt, J_{P}, _{H}=23.9 Hz, J=5.9 Hz, 1H), 1.16(t, J=7.0 Hz, 6H) ; ³¹P-NMR(CDCl₃) δ22.99(s);¹H-NMR(CD₃OD) δ7.31-7.16(m, 5H), 4.07-3.91(m, 4H), 3.2(dt, J=6.2 Hz, J_{P}, _{H}=16.6 Hz, 2H), 2.65(tt, J=6.2 Hz, J_{P}, _{H}=23.8 Hz, 1H), 1.20(t, J=7.0 Hz, 6H) ; ³¹P-NMR(CD₃OD) δ23.59(s) ; FAB-MS(m/z): (M⁺+H) 323

### (Compound 16)

The Compound 16 having the physical properties below was obtained from the Compound 15 by the same operations as in the preparation of the Compound 3.

### Compound 16

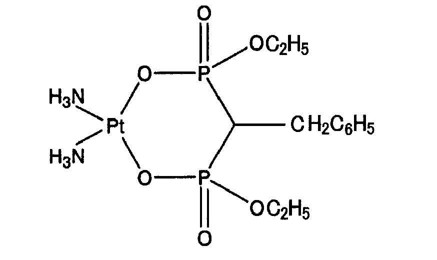

¹H-NMR(CD₃OD) δ7.35-7.08(m, 5H), 4.09-3.82(m, 4H), 3.24-3.06(m, 2H), 2.35(m, 1H), 1.20-1.04(m, 6H);³¹P-NMR(CD₃OD) δ31.54(s), 31.13(s); FAB-MS(m/z): (M⁺+H) 550

### (Example 7)

### (Compound 17)

Paraformaldehyde (2.60 g, 86.5 mmol) and diethylamine (1.27 g, 17.3 mmol) were added to absolute methanol (50 mL), and the resulting mixture was heated to become a transparent solution. After cooling to room temperature, tetraethyl methylenebisphosphonate (5.00 g, 17.3 mmol) was added to the solution, and the resulting mixture was heated to reflux for 40 hours. After cooling the mixture to room temperature, the solvent was evaporated under reduced pressure. Toluene (25 mL) was added thereto, and the solution was evaporated under reduced pressure again. This operation was repeated twice to remove methanol completely to obtain the Compound 17 having the physical properties below. This intermediate was not purified to be used in the next stage.

### Compound 17

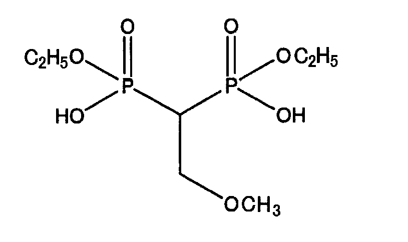

Colorless oil; ¹H-NMR(CDCl₃) δ4.23-4.14(m, 8H), 3.89(dt, J=5.5 Hz, J_{P}, _{H}=16.2 Hz, 2H), 2.69(tt, J_{P}, _{H}=23.9 Hz, J=5.5 Hz, 1H) 1.34 (t, J=6.9 Hz, 12H); ³¹P-NMR(CDCl₃) δ21.64(s)

### (Compound 18)

The Compound 17 was dissolved in anhydrous toluene (25 mL), and p-toluenesulfonic acid (15 mg) was added thereto, followed by heating the resulting mixture to reflux overnight. Methanol produced during the reaction was removed by Dean-Stark trap. After cooling to room temperature, the solvent was evaporated under reduced pressure. The residues were dissolved in chloroform (25 mL), and the resulting solution was washed with water (3×10 mL). The organic phase was dried over anhydrous sodium sulfate and the solvent was then evaporated. The residues were purified by silica gel chromatography (hexane:acetone= 1:1→5:95) to obtain the Compound 18 (3.47 g, 67%) having the physical properties below (see Charles R. Degenhardt, and Don C. Burdsall, J. Org. Chem., 1986, 51, 3488-3490). Compound 18 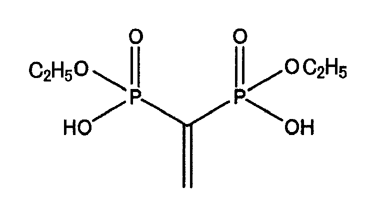 Colorless oil; ¹H-NMR(CDCl₃) δ6.99(dd, J_{P}, _{H}=33.9 Hz, 2H), 4.22-4.07(m, 8H), 1.36(t, J=7.1 Hz, 12H); ³¹P-NMR(CDCl₃) δ13.43(s)

### (Compound 19)

Under argon stream, 5% Pd-C (400 mg) and the Compound 17 (800 mg, 2.66 mmol) dissolved in ethanol (10 mL) were added to ethanol (40 mL), and the atmosphere was replaced with hydrogen, followed by stirring the resulting mixture at room temperature for 2 hours. The atmosphere was replaced with argon again, and the reaction solution was double-filtered, followed by evaporation of the obtained filtrate under reduced pressure to obtain the compound 19 (761 mg, 95%) having the physical properties below (see Sergio H. Szajnman, Andrea Montalvetti, Youhong Wang, RobertoDocampo, and Juan B. Rodriguez, Bioorg. Med. Chem. Lett., 2003, 13, 3231-3235).

### Compound 19

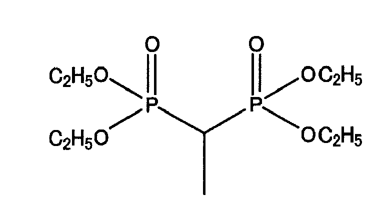

Colorless oil; ¹H-NMR(CDCl₃) δ4.18(dq, J_{P}, _{H}=8.6 Hz, J=7. 0 Hz, 8H), 2. 39 (qt, J_{P}, _{H}=23.6 Hz, J=7.3 Hz, 1H), 1. 47 (dt, J_{P}, _{H}=16.6 Hz, J=7.5 Hz, 3H), 1.34(t, J=7.0 Hz, 12H)

### (Compound 20)

The Compound 20 (84%, 2 or more stages) having the physical properties below was obtained from the Compound 19 by the same operations as in the preparation of the Compound 2.

### Compound 20

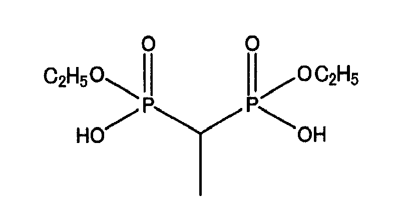

Colorless oil; ¹H-NMR(CDCl₃) δ6.11(br, 2H), 4.19-4.11(m, 4H), 2.40(qt, J=7.2 Hz, J_{P}, _{H}=23.9 Hz, 1H), 1.47(dt, J=7.2 Hz, J_{P}, _{H}=17.1 Hz, 3H), 1.33(t, J=7.1 Hz, 6H); ¹H-NMR(CD₃OD) δ4.12(dq, J_{P}, _{H}=8.0 Hz, J=7.0 Hz, 4H), 2.40(qt, J_{P}, _{H}=23.4 Hz, J=7.2 Hz, 1H), 1. 44 (dt, J_{P}, _{H}=17.2 Hz, J=7.3 Hz, 3H), 1.32 (t, J=7.0 Hz, 6_{H}); ³¹P-NMR(CD₃OD) δ24.19(s); FAB-MS (m/z): (M⁺+H) 246

### (Compound 21)

The Compound 21 having the physical properties below was obtained from the Compound 20 by the same operations as in the preparation of the Compound 3.

### Compound 21

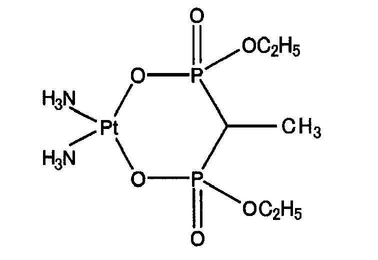

White powder; Yield: 14%;¹H-NMR(CD₃OD) δ4.01(dq, J=J_{P}, _{H}=7.0 Hz, 4H), 2.34-2.12(m, 1H), 1.53-1.35(m, 3H), 1.28(t, J=7.0 Hz, 6H);³¹P-NMR(CD₃OD) δ33.82(s);FAB-MS(m/z):(M⁺+H)474; Analysis: Calcd.: C₆H₂₀N₂O₆P₂Pt C:15.23, H:4.26, N:5.92; Found: C:14.85, H:4.19, N:6.03

### (Example 8)

### (Compound 22)

Under argon stream, to a solution of the Compound 14 (500 mg, 1.67 mmol) dissolved in 10 mL of THF, a solution of 2.2 M MeLi in diethylether (1.14 mL) was added dropwise at 0°C. After stirring the resulting mixture at 0°C for 2 hours, the mixture was allowed to warm to room temperature, and saturated aqueous ammonium chloride solution (20 mL) was added to the reaction solution. The reaction mixture was extracted with diethylether (3×20 mL). The organic phase was dried, and the solvent was then evaporated under reduced pressure. The residues were purified by silica gel chromatography (CH₂Cl₂:MeOH=98:2) to obtain the Compound 22 (305 mg, 58%) having the physical properties below (see Sergio H. Szajnman, Andrea Montalvetti, Youhong Wang, RobertoDocampo, and Juan B. Rodriguez, Bioorg. Med. Chem. Lett., 2003, 13, 3231-3235).

### Compound 22

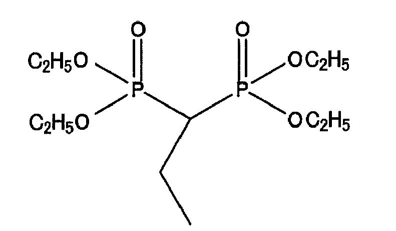

Colorless oil; ¹H-NMR(CDCl₃) δ4.22-4.14(m, 8H), 2.21(tt, J_{P}, _{H}=24.2 Hz, J=6.0 Hz, 1H), 2.07-1.91(m, 2H), 1.34(t, J=7.1 Hz, 12H), 1.16(t, J=7.5 Hz, 3H);³¹P-NMR(CDCl₃) δ24.44(s); FAB-MS (m/z) : (M⁺+H) 316

### (Compound 23)

The Compound 23 (94%, 2 or more stages) having the physical properties below was obtained from the Compound 22 by the same operations as in the preparation of the Compound 2.

### Compound 23

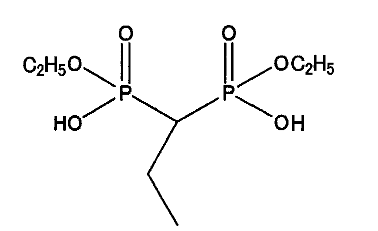

Colorless oil; ¹H-NMR(CDCl₃) δ5.42(br, 2H), 4.24-4.11(m, 4H), 2.19(tt, J_{P}, _{H}=24.6 Hz, J=5.4 Hz, 1H), 2.07-1.91(m, 2H), 1.33(t, J=7.1 Hz, 6H), 1.12(t, J=7.5 Hz, 3H);³¹P-NMR(CDCl₃) δ24.11(s); ¹H-NMR(CD₃OD) δ4.12(dq, J_{P}, _{H}=8.4 Hz, J=7.0 Hz, 4H), 2.20(tt, J_{P}, _{H}=24.1 Hz, J=6.0 Hz, 1H), 2.05-1.89(m, 2H), 1.32(t, J=7.0 Hz, 6H), 1.17(t, J=7.4 Hz, 3H);³¹P-NMR(CD₃OD) δ24.46(s); FAB-MS (m/z): (M⁺+H) 260

### (Compound 24)

The Compound 24 having the physical properties below was obtained from the Compound 23 by the same operations as in the preparation of the Compound 3.

### Compound 24

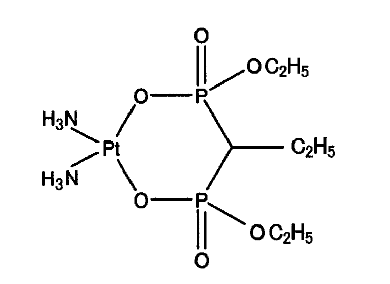

White powder; Yield: 5.8%;¹H-NMR(CD₃OD) δ4.00(dq, J=J_{P}, _{H}=7.1 Hz, 4H), 2.14-1.88(m, 3H), 1.28(t, J=7.1 Hz, 6H), 1.17(t, J=7.3 Hz, 3H);³¹P-NMR(CD₃OD) δ33.24(s);FAB-MS(m/z):(M⁺+H)488; Analysis: Calcd.: C₇H₂₂N₂O₆P₂Pt C:17.25, H:4.55, N:5.75; Found: C:16.95, H:4.40, N:5.80

### (Example 9)

### (Compound 25)

Benzyltributylammonium bromide (1.78 g, 5.00 mmol) was suspended in 50% aqueous sodium hydroxide solution, and tetraethyl methylenebisphosphonate (1.24 mL, 5.00 mmol) dissolved in 1,3-dibromopropane (15 mL) was added to this suspension. The reaction mixture was stirred vigorously at room temperature for 48 hours, and the mixture was then poured to CH₂Cl₂ (100 mL), followed by addition of water thereto until all the solids were dissolved. The organic phase was washed with water (6×100 mL) and saturated brine (2x100 mL). The organic phase was dried over anhydrous sodium sulfate and the solvent was then evaporated under reduced pressure. The residues were purified by silica gel chromatography (CH₂Cl₂:MeOH=99:1→95:5) to obtain the Compound 25 (0.64 g, 39%) having the physical properties below (see Jane R. Hanrahan, David W. Hutchinson, Tetrahedron Letters, 1993, 34(23), 3767-3768).

### Compound 25

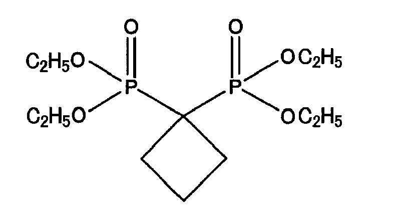

Colorless oil; ¹H-NMR(CDCl₃) δ4.23-4.16(m, 8H), 2.69-2.55(m, 4H), 2.24-2.16(m, 2H), 1.35(t, J=7.1 Hz, 12H); ³¹P-NMR(CDCl₃) δ26.72(s); FAB-MS (m/z): (M⁺+H) 329

### (Example 10)

### (Compound 26)

Under argon stream, methylbenzylamine (6.0 g, 50 mmol), diethylphosphite (21 g, 153 mmol) and triethylorthoformate (8.7 g, 59 mmol) were mixed together, and the resulting mixture was heated to reflux for 4 hours. After cooling the mixture to room temperature, it was extracted with chloroform, and the organic phase was washed with 1N sodium hydroxide three times, followed by its washing twice with saturated brine, drying over sodium sulfate and evaporation under reduced pressure. The obtained residues were purified by silica gel chromatography (hexane/ethyl acetate) to obtain the Compound 26 (12.1 g, 61%). [C-(N,N-methylbenzylamino)]methylenebis(diethylphosphonate)

### (Compound 26)

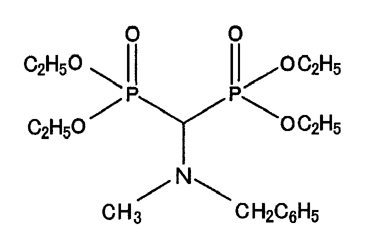

Colorless oil; ¹H-NMR(400MHz, CDCl₃) δ7.39-7.23(m, 5H), 4.26-4.10(m, 8H), 3.99(s, 2H), 3.49(t, J_{P}, _{H}=25.0 Hz, 1H), 2.66(t, J_{P}, _{H}=1.3 Hz, 3H), 1.35 (dt, J_{P}, _{H}=1.69 Hz, J=7.11 Hz, 12H); ³¹P-NMR(CDCl₃) δ19.50(S)

### (Compound 27)

Under argon stream, the Compound 26 (9.7 g, 24 mmol) and distilled morpholine (10.4 g, 119 mmol) were mixed together, and the resulting mixture was heated to reflux for 6 hours. After cooling the mixture to room temperature, it was evaporated under reduced pressure. The obtained residues were purified by silica gel chromatography (chloroform/methanol) to obtain the Compound 27 (9.4 g, 61%). [C-(N,N-methylbenzylamino)]methylenebis(ethylphosphonate) morpholinium salt (Compound 27) 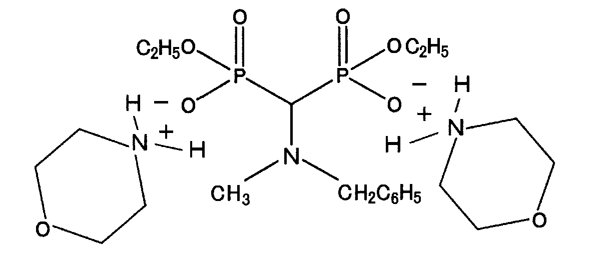 Yellow solid; ¹H-NMR (400MHz, CDCl₃) δ7.53-7.22(m, 5H), 4.20(s, 2H), 4.05-3.89(dm, 4H), 3.83(s, 4H), 3.35(t, J_{P}, _{H}=22 Hz, 1H) 3.10(s, 4H), 2.69(s, 3H), 1.27 (t, 6.9 Hz, 6H)

### (Compound 28)

Under argon stream, the Compound 27 (1.46 g, 2.7 mmol) was dissolved in methanol, 11 mL (20.9 mmol) of the Amberlite IR-120(H⁺) resin was added to the solution, and then the resulting mixture was left for 1.5 hours, following by the filtration to remove the resin. Thereafter, the filtrate was evaporated under reduced pressure to obtain the Compound 28 (733 mg, 75%).

### [C-(N,N-methylbenzylamino)]methylenebis(ethylphosphonic acid) (Compound 28)

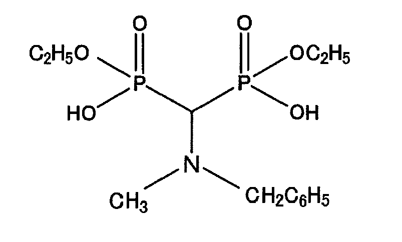

Yellow solid; ¹H-NMR (400MHz, CDCl₃) δ9.94(br, 2H), 7.72-7.39(m, 5H), 4.74(s, 2H), 4.09(s, 4H), 3.82(t, J_{P}, _{H}=19.7 Hz, 1H), 3.24(s, 3H), 1.27(t, 6H);³¹P-NMR(CDCl₃) δ8.14(s)

### (Compound 29)

Cisplatin (0.33 mmol) and silver nitrate (0.66 mmol) were dissolved in 5 mL of Milli-Q water, and the resulting solution was stirred in the dark at room temperature for 7 days. The obtained suspension was centrifuged at 4000 rpm for 5 minutes, and the supernatant was collected and filtered to obtain a transparent filtrate. To the obtained filtrate, 4 mL of a strongly basic ion-exchange resin Amberlite IRA(OH⁻) was added, and the resulting mixture was stirred at room temperature for 2 hours. After confirming with pH indicator paper that the solution became basic, the resin was removed, and the Compound 28 (0.33 mmol) was added to the filtrate, followed by 4 days of stirring in a water bath at 50°C in the dark. The obtained suspension was filtered and evaporated under reduced pressure. To the obtained residues, absolute methanol or absolute ethanol was added, and the obtained suspension was filtered, followed by evaporation of the filtrate under reduced pressure. The obtained residues were recrystallized with absolute methanol/anhydrous tetrahydrofuran, and the precipitated solids were filtered and dried in the air to obtain the Compound 29.

### Cis-diamine[C-(N,N-methylbenzylamino)methylenebis (ethylphosphonate)]platinum(II) (Compound 29)

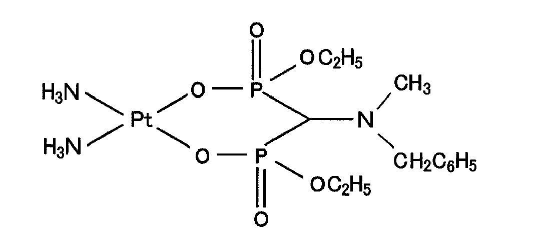

White solid; FAB-MS (m/z): (M⁺+H) 579

### (Example 11)

### (Compound 30)

By the same operations as in the preparation of the Compound 26, the Compound 30 (15.7 g, 79%) was obtained from ethylbenzylamine (6.4 g, 47 mmol), diethylphosphite (20 g, 147 mmol) and triethylorthoformate (8.5 g, 57 mmol).

### [C-(N,N-ethylbenzylamino)]methylenebis(diethylphosphonate) (Compound 30)

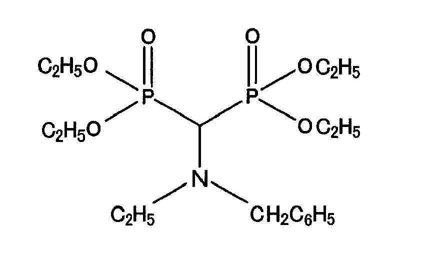

Colorless oil; ¹H-NMR (400MHz, CDCl₃) δ7.41-7.21(m, 5H), 4.24-4.08(m, 8H), 4.04(s, 2H), 3. 55 (t, J_{P}, _{H}=25.0 Hz, 1H), 2.97(q, 2H), 1.33(t, 12H), 1.07(t, 3H); ³¹P-NMR(CDCl₃) δ19.9(s)

### (Compound 31)

By the same operations as in the preparation of the Compound 27, the Compound 31 (60%) was obtained from the Compound 30.

### [C-(N,N-ethylbenzylamino)]methylenebis (ethylphosphonate)morpholinium salt (Compound 31)

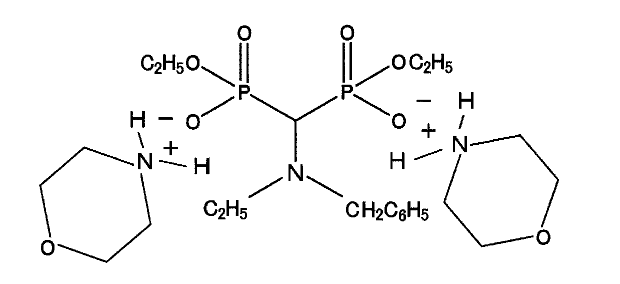

Yellow solid; ¹H-NMR (400MHz, CDCl₃) δ7.49-7.22(m, 5H), 4.24 (s, 2H), 4.1-3.87(m, 4H), 3. 82 (t, 4H), 3. 39 (t, J_{P}, _{H}=22 Hz, 1H), 3.07(t, 4H), 1.28 (t, 6H), 1.08(t, 3H)

### (Compound 32)

By the same operations as in the preparation of the Compound 28, the Compound 32 (76%) was obtained from the Compound 31.

### [C-(N,N-ethylbenzylamino)]methylenebis(ethylphosphonic acid) (Compound 32)

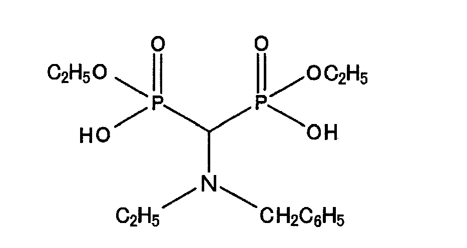

Brown solid; ¹H-NMR (400MHz, CDCl₃) δ10.1(br, 2H), 7.77-7.35(m, 5H), 4.72(s, 2H), 3.96(q, J=6.9 Hz, 4H), 3.79(q, J=7.1 Hz, 2H), 3.46(t, J_{P}, _{H}=19.1 Hz, 1H), 1.49(t, J=7.1 Hz, 3H), 1.20(t, 6.9 Hz, 6H); ³¹P-NMR(CDCl₃) δ8.23(s); FAB-MS (m/z) (M⁺) 365

### (Compound 33)

By the same operations as in the preparation of the Compound 29, the Compound 33 was obtained from the Compound 32.

### Cis-diamine[C-(N,N-ethylbenzylamino)methylenebis (ethylphosphonate)]platinum(II) (Compound 33)

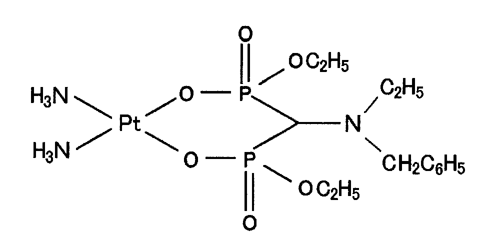

White solid; FAB-MS (m/z): (M⁺+H) 593

### (Example 12)

### (Compound 34)

By the same operations as in the preparation of the Compound 26, the Compound 34 (14.1 g, 45%) was obtained from dibenzylamine (15.4 g, 78 mmol), diethylphosphite (33.4 g, 242 mmol) and triethylorthoformate (13.9 g, 93.6 mmol).

### [C-(N,N-dibenzylamino)]methylenebis(diethylphosphonate) (Compound 34)

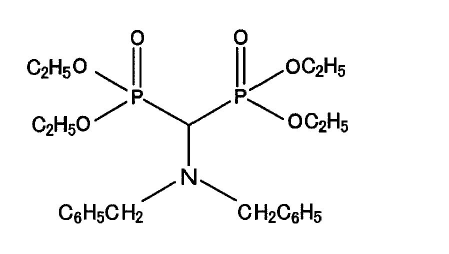

White solid; ¹H-NMR (400MHz, CDCl₃) δ7.40-7.21(m, 1H), 4.21-4.10(m, 4H), 4.09-4.01(m, 8H), 3.55(t, J_{P}, _{H}=25.1 Hz, 1H), 1.32(dt, J_{P}, _{H}=2.23 Hz, J=6. 92 Hz, 12H) ; ³¹P-NMR(CDCl₃) δ20.08(s)

### (Compound 35)

By the same operations as in the preparation of the Compound 27, the Compound 35 (62%) was obtained from the Compound 34.

### [C-(N,N-dibenzylamino)]methylenebis (ethylphosphonate)morpholinium salt (Compound 35)

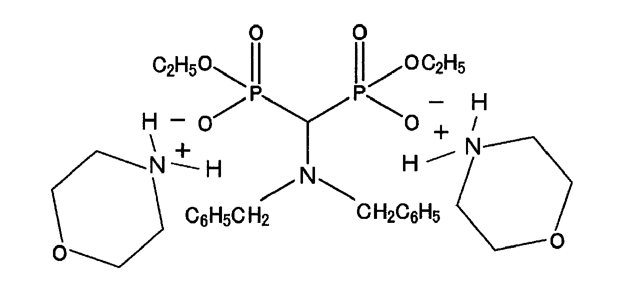

White solid; ¹H-NMR(400MHz, CDCl₃) δ7.52-7.18(m, 10H), 4.10 (s, 4H), 3.85(m, 2H), 3.64(m, 2H), 3.76(t, J=4.49 Hz, 4H), 3.29(t, J_{P}, _{H}=22.5 Hz, 1H), 3.00(t, J=4.87 Hz, 4H), 1.25 (t, J=6.93 Hz, 6H)

### (Compound 36)

By the same operations as in the preparation of the Compound 28 except the reaction time (19 hours), the Compound 36 (90%) was obtained from the Compound 35.

### [C-(N,N-dibenzylamino)]methylenebis(ethylphosphonic acid) (Compound 36)

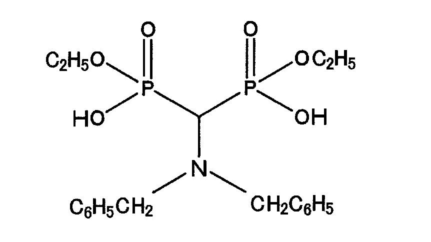

White solid; ¹H-NMR(400MHz, CDCl₃) δ7.45-7.26(m, 10H), 6.98(br, 2H), 4.42(s, 4H), 4.03-3.96(m, 4H), 3.51(t, J_{P}, _{H}=21.2 Hz, 1H), 1.28(t, J=7.11 Hz, 6H); ³¹P-NMR(CDCl₃) δ13.00(s); FAB-MS (m/z) (M⁺) 427

### (Compound 37)

By the same operations as in the preparation of the Compound 29, the Compound 37 having the physical properties below was obtained from the Compound 36.

### Cis-diamine[C-(N,N-dibenzylamino)methylenebis (ethylphosphonate)]platinum(II) (Compound 37)

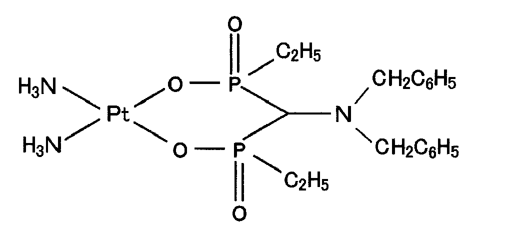

White solid; ³¹P-NMR(CD₃OD) δ29.23(s) ;FAB-MS (m/z) : (M⁺+H) 655

### (Example 13)

### (Compound 38)

Diethylphosphite (0.69 g, 5 mmol) was added to anhydrous acetonitrile (8 mL), and the resulting mixture was stirred to dissolve the solid. To this solution, trichloroisocyanuric acid (0.58 g, 2.5 mmol) dissolved in 4 mL of anhydrous acetonitrile was added, and the resulting mixture was stirred under argon stream at room temperature for 4 hours. The precipitated white solids were removed by filtration, and the solvent was evaporated under reduced pressure. These residues were purified by distillation to obtain the Compound 38 (0.70 g, 81%).

### Diethylchlorophosphate (Compound 38)

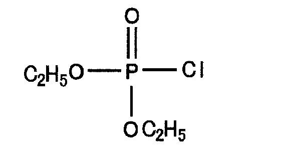

Colorless oil; ¹H-NMR(CDCl₃) δ4.35-4.21(m, 4H), 1.41(dt, J_{P}, _{H}=1.12 Hz, J=7.11 Hz, 6H);³¹P-NMR(CDCl₃) δ4.88(s)

### (Compound 39)

To dichloromethane (5 mL), benzylamine (0.35 g, 3.3 mmol) and triethylamine (0.36 g, 3.6 mmol) were added, and the resulting mixture was stirred under argon stream, followed by cooling thereof to 0°C. To this solution, the Compound 38 (0.52 g, 3 mmol) was added dropwise, and the resulting mixture was stirred at room temperature for 3 hours. To the reaction solution, dichloromethane (30 mL) was added, and the resulting mixture was washed with water, saturated aqueous sodium hydrogen carbonate solution and saturated brine, followed by drying the mixture over sodium sulfate and evaporation thereof under reduced pressure. The resulting residues were purified by distillation to obtain the Compound 39 (0.71 g, 98%).

### Diethyl N-benzylphosphoraimidate (Compound 39)

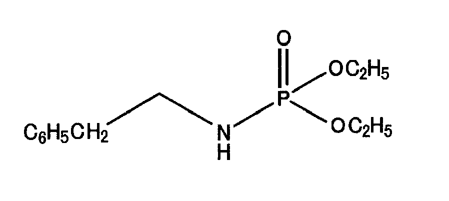

Pale yellow oil; ¹H-NMR(CDCl₃) δ7.33(d, 3H), 7.28-7.24(m, 1H), 4.13-3.98(m, 4H), 1.31(dt, J_{P}, _{H}=0.75 Hz, J=7.11 Hz, 6H); ³¹P-NMR(CDCl₃) δ8.88(s)

### (Compound 40)

A solution (5.2 mL) of 1M lithium bis(trimethylsilyl) amide in THF (tetrahydrofuran) was cooled to -78°C, and the Compound 39 (0.63 g, 2.6 mmol) mixed with THF (3 mL) was added to the solution, followed by stirring the resulting mixture for 30 minutes. To this solution, diethylchlorophosphate (0.45 g, 2.6 mmol) mixed with THF (3 mL) was added dropwise and the resulting mixture was stirred for 1 hour, followed by stirring the mixture for 16 hours while allowing it to warm gradually to room temperature. Water (20 mL) was added to the reaction solution, and the mixture was stirred, followed by 3 times of extraction with dichloromethane (50 mL). The organic phase was washed with aqueous 0.5 M hydrochloric acid solution, saturated aqueous sodium hydrogen carbonate solution and saturated brine, and dried over sodium sulfate, followed by evaporation under reduced pressure. The residues were purified by silica gel chromatography (hexane/ethyl acetate) to obtain the Compound 40 (0.40 g, 35%).

### N-benzylimidobis(diethylphosphonate) (Compound 40)

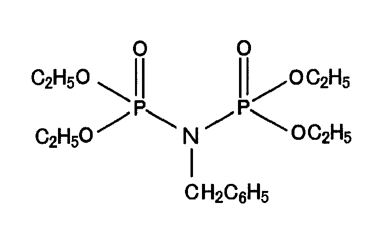

Colorless oil; ¹H-NMR(CDCl₃) δ7.53(d, J=6.9 Hz, 2H), 7.31(t, J=7.2 Hz, 2H), 7.25(t, J=7. 3 Hz, 1H), 4.56(t, J_{P}, _{H}=13.7 Hz, 2H), 4.14-4.04(m, 4H), 4.02-3.93(m, 4H), 1.26(t, J=7.0 Hz, 12H); ³¹P-NMR(CDCl₃) δ3.92(s)

### (Compound 41)

The Compound 40 (0.37 g, 0.98 mmol) was dissolved in morpholine (5 mL), and the resulting solution was stirred at 100°C for 16 hours. After cooling the solution to room temperature, unreacted morpholine was evaporated under reduced pressure. The residues were washed with anhydrous ether (20 mL) and dried under reduced pressure. The residues were purified by silica gel chromatography (methanol/chloroform) to obtain the Compound 41 (0.33 g, 83%).

### N-benzylimidobis(ethylphosphonate)morpholinium salt (Compound 41)

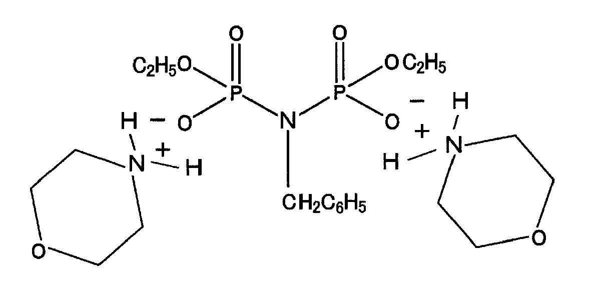

Colorless oil; ¹H-NMR(CDCl₃) δ7.58(d, J=7.7 Hz, 2H), 7.26(d, J=7.0 Hz, 2H), 7.13(t, J=7. 3 Hz, 1H), 4.52(t, J_{P}, _{H}=13.3 Hz, 2H), 3.89-3.86(m, 4H), 3.76(s, 8H), 2.89(s, 8H), 1.19(t, J=7.1 Hz, 6H); ³¹P-NMR(CDCl₃) δ3.58(s)

### (Compound 42)

Amberlite IR-120(H⁺) resin (5.00 g) was added to 20 mL of methanol, and the Compound 41 (0.33 g, 0.81 mmol) was further added thereto, followed by stirring the resulting mixture at room temperature for 24 hours. This solution was filtered, and the solvent was evaporated under reduced pressure to obtain the Compound 42 (0.18 g, 64.9%).

### N-benzylimidobis(ethylphosphonic acid) (Compound 42)

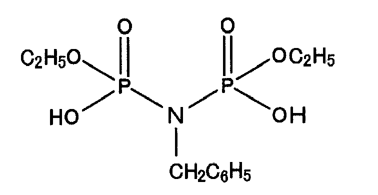

White solid; ¹H-NMR(D₂O) δ7.38(d, J=7.5 Hz, 2H), 7.30(t, J=7.5 Hz, 2H), 7.23(t, J=7.2 Hz, 1H), 4.40(t, J_{P}, _{H}=13.8 Hz, 2H), 3.86-3.79(m, 4H), 1.08(t, J=7.1 Hz, 6H); ³¹P-NMR(D₂O) δ3.95(s)

### (Compound 43)

Cisplatin (0.15 g, 0.5 mmol) and silver oxide (0.12 g, 0.5 mmol) were dissolved in DMF (3 mL), and the Compound 42 (0.16 g, 0.5mmol) was added to the solution, followed by stirring the resulting mixture at room temperature for 4 hours. The white precipitates were removed by filtration through Celite, and the solvent was evaporated under reduced pressure. The residues were recrystallized with absolute ethanol to obtain the Compound 43 (0.063 g, 23%).

### Cis-diamine[N-benzylimidobis(ethylphosphonate)]platinum(II) (Compound 43)

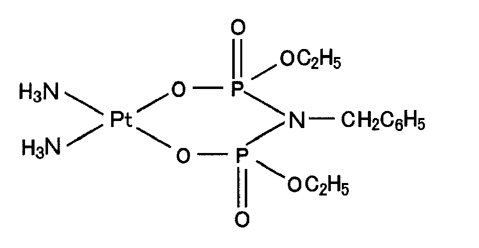

White solid; ¹H-NMR(D₂O) δ7.51(d, J=7.7 Hz, 2H), 7.38(t, J=7. 5 Hz, 2H), 7.29 (t, J=7.3 Hz, 1H), 4. 44 (t, J_{P}, _{H}=14.0 Hz, 2H), 3.94-3.86(m, 4H), 1.18(t, J=7.0 Hz, 6H);³¹P-NMR(D₂O) δ12.25(s); FAB-MS (m/z): (M⁺+H) 551

The present invention will be described more concretely by way of Experiment Examples below. The compound of the present invention is not limited to the Experiment Examples below.

### (Experiment Example 1)

The adsorption action of the platinum complex of the Compound 3 to hydroxyapatite was investigated.

To 2 mL of HEPES buffer, 5,10,20,40 or 80 mg of hydroxyapatite (Bio-Rad, Hydroxyapatite, typeI, 40 µm) was added, and the resulting mixture was shaken for 24 hours at 37°C. Thereafter, 100 µL of the platinum complex of the Compound 3 was added to each HEPES buffer, and the resulting mixture was shaken for 1.5 hours at 37°C. The solution was filtered, and phosphorus (P) was measured by ³¹P-NMR (Varian VXR-300S) and platinum was measured by an atomic absorption photometer (Hitachi Z-5710 AAS).

The absorption to hydroxyapatite, which was calculated from the results of measurement by ³¹P-NMR and atomic absorption photometry, increased dependently on the amount of hydroxyapatite and was 20%, 40%, 55%, 70% and 75%, respectively. From these results, it was shown that the platinum complex of the Compound 3 adsorbs to hydroxyapatite.

### (Experiment Example 2)

The activity of the platinum complex of the Compound 3 to suppress the bone resorptive activity by osteoclasts was investigated.

From long bone of a Japanese white rabbit of 7 days old, unfractionated total bone cells were prepared, and the cells were dispersed on collagen gel, followed by treatment with various enzyme treatments to prepare a cell suspension containing only osteoclasts. The isolated osteoclasts were dispersed on ivory pieces, and the platinum complex of the Compound 3 was added thereto at final concentrations of 1, 10, 100 nM and 1 µM. The calculation was carried out based on the area of resorption lacunae formed after 24 hours of culture. As a result, the platinum complex of the Compound 3 suppressed the area of the formed resorption lacunae at the ratios of 3%, 20%, 35% and 65% dependently on its concentration.

### (Experiment Example 3)

Using nude mice transplanted with human tumors SC-6-JCK and Lu-24, the anti-tumor action of the substance having the structure below (Compound 44), among the compounds of the present invention, was investigated. Each of SC-6-JCK and Lu-24 was subcutaneously implanted in nude mice, and, when the tumor volumes reached to 100 to 300 mm³, 7.0 mg and 10.0 mg/kg of the present test substance were respectively administered to the SC-6-JCK tumor-bearing mice, and 10.0 mg and 12.0 mg/kg of the present test substance were respectively administered to the Lu-24 tumor-bearing mice, from the tail vein 3 times intermittently at 4 days' intervals. As a result, the action of suppression of the tumor volume was observed in a dose-dependent manner. Further, BUN (blood urea nitrogen) was measured 4 days after completion of the administration as an index of nephrotoxicity, but an increase of the BUN value was not observed, so that nephrotoxicity was not observed.

### Compound 44

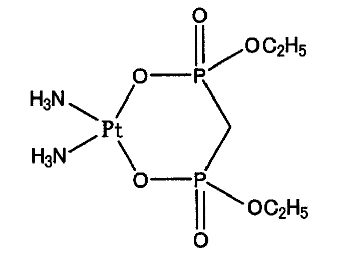

### (Experiment Example 4)

Using cultured human cancer cells, the activity of the substance having the structure below (Compound 45), among the compounds of the present invention, to suppress the growth of human cancer cells was investigated. Cancer cells were dispersed on a 96-well plate, and, 24 hours later, the test drug was added thereto at a concentration within the range of 1.0×10⁻⁴ to 1.0×10⁻⁸ M, followed by 48 hours of culture and measurement of the cell growth by colorimetry using sulfo rhodamine B.

### Compound 45

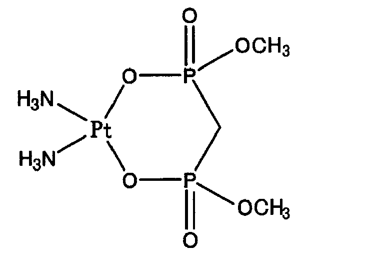

The observed IC₅₀ was within the range of 1.5×10⁻⁶ to 1.0×10⁻⁴ M in cancer cells of each of lung cancer, gastric cancer, colon cancer, ovarian cancer, brain tumor, breast cancer, renal cancer, prostate cancer and melanoma.

### (Experiment Example 5)

Using cultured human cancer cells, the activity of the substance having the structure below (Compound 46), among the compounds of the present invention, to suppress the growth of human cancer cells was investigated.

### Compound 46

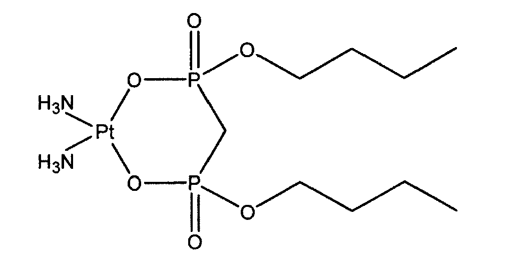

The observed IC₅₀ value was within the range of 5.4×10⁻⁶ to 1.0×10⁻⁴ M in cancer cells of each of lung cancer, gastric cancer, colon cancer, ovarian cancer, brain tumor, breast cancer, renal cancer, prostate cancer and melanoma.

### (Experiment Example 6)

Using cultured human cancer cells, the activity of the substance having the structure below (Compound 47), among the compounds of the present invention, to suppress the growth of human cancer cells was investigated.

### Compound 47

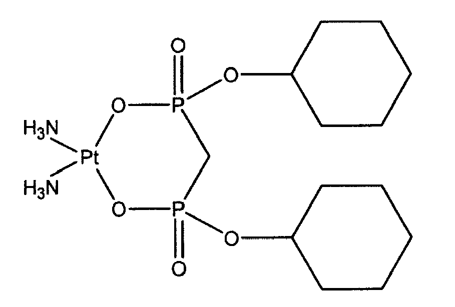

The observed IC₅₀ value was within the range of 1.5×10⁻⁵ to 1.0×10⁻⁴ M in cancer cells of each of lung cancer, gastric cancer, colon cancer, ovarian cancer, brain tumor, breast cancer, renal cancer, prostate cancer and melanoma.

The pharmaceutical of the present invention exhibited a high degree of adsorption to bone and is an agent for therapy of cancers, which agent is capable of exerting strong effects against primary carcinomas and cancers metastasized to bone.

## Claims

1. A metal complex compound represented by the following General Formula (1): 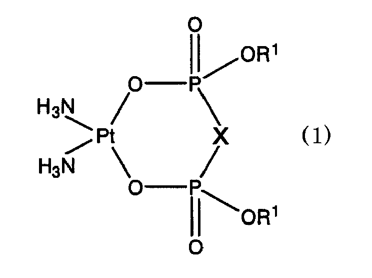 (wherein
R¹ independently represents C₁-C₁₀ alkyl which may be branched or have a substituent; or a C₃-C₃₀ cyclic group which may have a substituent; and
X represents CHR² (wherein R² represents a hydrogen atom; C₁-C₁₀ alkyl which may be branched or have a substituent; a C₃-C₃₀ cyclic group which may have a substituent; or NR³R⁴ (wherein R³ and R⁴ independently represent C₁-C₁₀ alkyl which may be branched or have a substituent; or a C₃-C₃₀ cyclic group which may have a substituent)), an oxygen atom or NR⁵ (wherein R⁵ represents C₁-C₁₀ alkyl which may be branched or have a substituent; or a C₃-C₃₀ cyclic group which may have a substituent)).

2. The metal complex compound according to claim 1, wherein R¹ in said General Formula (1) is methyl, ethyl, propyl, butyl, octyl, cyclohexyl, phenyl or benzyl.

3. The metal complex compound according to claim 1, wherein said X is CHR² (wherein R² has the same meaning as described above).

4. The metal complex compound according to claim 3, wherein said R² in said General Formula (1) is a hydrogen atom, methyl, ethyl, propyl, butyl, octyl, benzyl or NR³R⁴ (wherein R³ and R⁴ independently represent a hydrogen atom, methyl, ethyl, propyl, butyl, octyl or benzyl).

5. The metal complex compound according to claim 1, wherein said X is an oxygen atom.

6. The metal complex compound according to claim 1, wherein said X is NR⁵ (wherein R⁵ has the same meaning as described above).

7. The metal complex compound according to claim 6, wherein the R⁵ in said General Formula (1) is a hydrogen atom, methyl, ethyl, propyl, butyl or octyl.

8. A therapeutic agent composition for a cancer, comprising as an active ingredient the metal complex compound according to claim 1 or a physiologically acceptable salt thereof.

9. The therapeutic agent composition for a cancer according to claim 8, which cures a primary carcinoma selected from the group consisting of lung cancer, gastric cancer, colon cancer, ovarian cancer, brain tumor, breast cancer, renal cancer, prostate cancer and melanoma.

10. The therapeutic agent composition for a cancer according to claim 8, which is a therapeutic agent for a metastasized cancer which cures a cancer metastasized to bone, whose primary carcinoma is selected from the group consisting of lung cancer, gastric cancer, colon cancer, ovarian cancer, brain tumor, breast cancer, renal cancer, prostate cancer and melanoma.

11. The therapeutic agent composition for a cancer according to claim 8, which is an oral preparation.

12. The therapeutic agent composition for a cancer according to claim 9, which is an oral preparation.

13. The therapeutic agent composition for a cancer according to claim 10, which is an oral preparation.

14. An intermediate for a metal complex compound represented by the following General Formula (2): 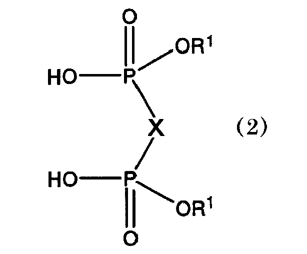 (wherein
R¹ independently represents C₁-C₁₀ alkyl which may be branched or have a substituent; or a C₃-C₃₀ cyclic group which may have a substituent; and
X represents CHR² (wherein R² represents a hydrogen atom; C₁-C₁₀ alkyl which may be branched or have a substituent; a C₃-C₃₀ cyclic group which may have a substituent; or NR³R⁴ (wherein R³ and R⁴ independently represent C₁-C₁₀ alkyl which may be branched or have a substituent; or a C₃-C₃₀ cyclic group which may have a substituent)), an oxygen atom or NR⁵ (wherein R⁵ represents C₁-C₁₀ alkyl which may be branched or have a substituent; or a C₃-C₃₀ cyclic group which may have a substituent)).
